# EUROPEAN PATENT SPECIFICATION

(11) **EP 4 005 550 B1**
(45) Date of publication and mention of the grant of the patent: **07.05.2025**
(21) Application number: 19938137.7
(22) Date of filing: 25.07.2019
(51) Int. Cl.: A61K 8/86, A61Q 19/02, A61Q 5/00, A61Q 1/12, A61K 8/89, A61K 8/44, A61K 8/39, A61K 8/19, A61K 8/02, A61K 8/27, A61K 8/29, A61K 8/58

(54) **HYDROPHILIZED INORGANIC POWDER AND COSMETIC COMPOUNDED WITH SAID HYDROPHILIZED INORGANIC POWDER**
HYDROPHILISIERTES ANORGANISCHES PULVER UND MIT DEM HYDROPHILISIERTEN ANORGANISCHEN PULVER ZUSAMMENGESETZTES KOSMETIKUM
POUDRE INORGANIQUE HYDROPHILISÉE ET PRODUIT COSMÉTIQUE MÉLANGÉ AVEC LADITE POUDRE INORGANIQUE HYDROPHILISÉE

(43) Date of publication of application: 01.06.2022
(73) Proprietor: Miyoshi Kasei, Inc., Tokyo 102-0073 (JP)
(72) Inventor: HASEGAWA, Yukio, Tokyo 102-0073 (JP)
(74) Representative: Glawe, Delfs, Moll
(86) International application number: PCT/JP2019/029294
(87) International publication number: WO 2021/014655

(56) References cited:
- EP-A1- 1 914 264
- WO-A1-2007/007521
- JP-A- 2002 080 748
- JP-A- 2002 080 748
- JP-A- 2012 219 028
- JP-A- 2013 071 920
- JP-A- 2015 010 095
- JP-A- 2015 010 095
- JP-A- 2016 117 698
- JP-A- 2017 081 848
- JP-A- 2017 081 848
- US-A1- 2006 013 843

## Description

### TECHNICAL FIELD

The present invention relates to a hydrophilized inorganic powder, and a cosmetic including the hydrophilized inorganic powder.

### BACKGROUND ART

In a cosmetic, a pigment or a UV scattering agent containing zinc oxide, titanium oxide, or the like as a base material is blended. There is a demand for blending the pigment or the UV scattering agent in an aqueous layer of an emulsion cosmetic, but zinc oxide or titanium oxide itself shows strong aggregability, and gives a powdery sensation and physical irritation to the skin, and therefore is generally subjected to a surface treatment.

In the light of the demand for blending in the aqueous layer, it is conceivable that the surface of zinc oxide is coated with a hydrophilization treatment agent (for example, silica), but zinc oxide having a surface coat of silica is eluted by reacting with an acid or an alkali blended in a cosmetic (see Cited Document 1). Therefore, in Cited Document 1, first, a hydrophobic first coat is formed on the surface of zinc oxide with a hydrophobization treatment agent (octyltriethoxysilane), and subsequently, a hydrophilic second coat is formed from a surfactant (emulsifier: PEG-11 methyl ether dimethicone) (see Cited Document 1). As a similar technology, Cited Document 2 discloses a technology in which titanium dioxide is first subjected to a hydrophobization treatment with sodium stearate and subsequently subjected to a hydrophilization treatment with isodecyl alcohol 6-ethoxylate or the like. Cited document 3 discloses the coating of a powder comprising a silicone resin and/or an organic powder containing a metal oxide and/or a metal hydroxide with a specific hydrophilizing agent, wherein the hydrophilizing agent includes water-soluble polyoxyalkylene glycol derivate.

### CITATION LIST

### PATENT LITERATURE

[Patent Literature 1] JP2016-222589A
[Patent Literature 2] JP4157039B
[Patent Literature 3] EP 1 914 264 A1

### SUMMARY

### TECHNICAL PROBLEM

The following analysis was conducted from the viewpoint of the present invention. The disclosures of the above prior art documents shall be incorporated into this document by reference.

As disclosed in Cited Documents 1 and 2, a wide variety of combinations of a hydrophobization treatment agent and a hydrophilization treatment agent can be selected, but there is a problem that desired properties cannot be achieved depending on the combination thereof. Here, there is no knowledge that can guide the selection of a hydrophobization treatment agent and a hydrophilization treatment agent that can achieve the desired properties.

Further, the blending amount of the hydrophilization treatment agent (that is, the surfactant) is preferably as small as possible from the viewpoint of irritation to the skin, but in Cited Document 1, the amount of PEG-11 methyl ether dimethicone with respect to coated zinc oxide is 10 wt%, and in Cited Document 2, with respect to 150 g of titanium dioxide, 18 g of isodecyl alcohol 6-ethoxylate and 12 g of cetyl alcohol 10-ethoxylate are used, and there is room for improvement.

Therefore, an object of the present invention is to contribute to the provision of a hydrophilized inorganic powder having favorable properties while reducing the blending amount of a surfactant, and a cosmetic including the hydrophilized inorganic powder.

### SOLUTION TO PROBLEM

According to a first aspect of the present invention,
a hydrophilized inorganic powder including an inorganic powder as a base material, a hydrophobic coat that covers the surface of the inorganic powder, and a hydrophilic coat that covers the hydrophobic coat, wherein
the composition of the hydrophilic coat is a nonionic surfactant(s) having a hydrophilic moiety and a carbon chain moiety,
the carbon chain moiety of the nonionic surfactant(s) has a branched structure sufficient for imparting self-dispersibility to the inorganic powder having hydrophobicity, the nonionic surfactant(s) is/are one or more selected from the following materials: polyoxyethylene (10) isostearyl ether; polyoxyethylene (10) isododecyl ether, polyoxyethylene (12) isostearate; polyoxyethylene (8) glyceryl isostearate; polyoxyethylene (20) glyceryl triisostearate; polyoxyethylene (20) octyldodecyl ether; polyoxyethylene (5) hexyldecyl ether; and polyglyceryl (10) monoisostearate, and the composition of the hydrophobic coat is one or more selected from octyltriethoxysilane, disodium stearoyl glutamate, hydrogen dimethicone, dimethylpolysiloxane, and methyl hydrogen polysiloxane. is provided.

According to a second aspect of the present invention, a cosmetic including the hydrophilized inorganic powder is provided.

### ADVANTAGEOUS EFFECTS OF INVENTION

According to each aspect of the present invention, a technology that contributes to the provision of a hydrophilized inorganic powder having favorable properties while reducing the blending amount of a surfactant(s), and a cosmetic including the hydrophilized inorganic powder is provided. Note that as the hydrophilized inorganic powder obtained according to the present invention, a powder for a cosmetic, a base material for a powder cosmetic, and the like are also considered.

### BRIEF DESCRIPTION OF THE DRAWINGS

[FIG. 1] FIG. 1 shows an example of an evaluation result of hydrophilicity and self-dispersibility.
[FIG. 2] FIG. 2 shows an example of an evaluation result of hydrophilicity and self-dispersibility.

### MODES

First, terms used in the present application will be explained.

### [Inorganic Powder]

The inorganic powder is a powder that serves as a base of the hydrophilized inorganic powder, and is preferably a powder composed of particles of a metal oxide or a metal hydroxide containing at least one of Ti, Zn, Si, Al, Fe, Mg, and Ce. Concrete examples thereof include titanium oxide, zinc oxide, silica, aluminum oxide, iron oxide, iron hydroxide, magnesium oxide, and cerium oxide. The inorganic oxide powder particles may be coated with another oxide or hydroxide.

In the present invention, the inorganic powder is not particularly limited as long as it is a powder used for ordinary cosmetics. That is, the inorganic powder also includes cericite, natural mica, calcined mica, synthetic mica, synthetic sericite, alumina, mica, talc, kaolin, bentonite, smectite, calcium carbonate, magnesium carbonate, magnesium silicate, aluminum silicate, calcium phosphate, silicic anhydride, magnesium oxide, barium sulfate, magnesium aluminometasilicate, iron oxide, chromium oxide, titanium oxide, zinc oxide, cerium oxide, aluminum oxide, magnesium oxide, Prussian blue, ultramarine, calcium carbonate, magnesium carbonate, calcium phosphate, aluminum hydroxide, magnesium sulfate, silicic acid, magnesium aluminum silicate, calcium silicate, barium silicate, strontium silicate, silicon carbide, a metal tungstate, magnesium aluminate, magnesium aluminometasilicate, chlorohydroxyaluminum, clay, zeolite, hydroxyapatite, a ceramic powder, aluminum nitride, silicon carbide, cobalt titanate, lithium cobalt titanate, cobalt aluminate, an inorganic blue pigment, low-order titanium oxide, fine particle titanium oxide, butterfly-shaped barium sulfate, petal-shaped zinc oxide, hexagonal plate-shaped zinc oxide, tetrapot-shaped zinc oxide, fine particle zinc oxide, titanium oxide-coated mica, titanium oxide-coated silica, titanium oxide-coated synthetic mica, titanium oxide-coated talc, fish scale foil, titanium oxide-coated colored mica, titanium oxide-coated borosilicate (sodium/calcium), titanium oxide-coated borosilicate (calcium/aluminum), red iron oxide-coated mica, red iron oxide-coated mica titanium, red iron oxide/black iron oxide-coated mica titanium, carmine-coated mica titanium, carmine/Prussian blue-coated mica titanium, mango violet, cobalt violet, a glass fiber, an alumina fiber, and the like.

### [Hydrophobic Coat and Hydrophobic Inorganic Powder]

The hydrophobic coat means a coat that is hydrophobic and covers the surface of the powder (also referred to as a hydrophobic first coat), and in the present application, the inorganic powder covered with the hydrophobic coat is referred to as a hydrophobic inorganic powder. Here, the hydrophobic coat is formed from an organic surface treatment agent, and therefore, the composition of the hydrophobic coat can be said to be an organic surface treatment agent.

The hydrophobic inorganic powder of the present invention is an inorganic powder having hydrophobicity. As an evaluation method, 20 g of purified water and 20 cc of isododecane are placed in a 50 cc glass vial, and 0.2 g of the powder is added thereto, and the vial is vigorously shaken up and down 10 times by hand at a shaking width of about 30 cm. The vial is left to stand in a constant temperature bath at 50°C for 3 days, and then taken out and vigorously shaken 10 times by hand in the same manner as described above. The powder is preferably such that the powder particles do not transfer to the aqueous layer after the vial is left to stand for 3 minutes.

### [Organic Surface Treatment Agent]

The organic surface treatment agent is an organic treatment agent for coating the surface of the inorganic powder to make it hydrophobic, and is also referred to as a hydrophobization treatment agent. As the organic surface treatment agent, one or more types of compounds selected from a silicone compound, an alkyl silane, an alkyl titanate, a polyolefin, an acylated amino acid, a fatty acid, lecithin, an ester oil, a fructooligosaccharide, an acrylic polymer, and a urethane polymer are exemplified.

As the silicone compound, methyl hydrogen polysiloxane (Shin-Etsu Chemical Co., Ltd.: KF99P, KF9901, X-24-9171, X-24-9221, or the like), dimethiconol, one-terminal alkoxysilyl dimethylpolysiloxane, trimethylsiloxysilicate, a cyclic methylhydrogen silicone such as tetrahydro tetramethylcyclotetrasiloxane, an acrylic silicone, a silicone acrylic, an amino-modified silicone, a carboxy-modified silicone, a phosphate-modified silicone, or the like can also be used. Other than these, as a commercially available one from Shin-Etsu Chemical Co., Ltd., KF-9908 (triethoxysilylethyl polydimethylsiloxyethyl dimethicone), KF-9909 (triethoxysilylethyl polydimethylsiloxyethyl hexyl dimethicone), or the like can also be used. As a representative silicone compound, hydrogen dimethicone, dimethylpolysiloxane, and methyl hydrogen polysiloxane are exemplified.

Examples of the alkyl silane include an alkyl alkoxy silane. Examples of the length of the alkyl chain include 1 to 18 carbon atoms, and concrete examples thereof include methyltriethoxysilane, octyltriethoxysilane, octadecyltriethoxysilane, aminopropyltriethoxysilane, and the like.

Examples of the alkyl titanate (organic titanate) include one having a Ti(OR₁)₄ structure as a basic skeleton, wherein R₁'s are independent of one another and is an alkyl group or an organic carbonyl group. As a generally available one, isopropyl triisostearoyl titanate (Plenact TTS; Ajinomoto Fine-Techno Co., Inc.), and the like are exemplified.

As the polyolefin, polyolefin resins having at least one carboxyl group in the molecule such as polyethylene and polypropylene can be exemplified. For example, low molecular weight polyethylene having a molecular weight of 500 to 40,000 and a melting point of 40°C or higher described in JP-A-63-179972, polyethylene oxide obtained by oxidizing polypropylene, maleated polyethylene, polypropylene oxide, and the like are exemplified.

As the acylated amino acid, an acylated compound of a saturated fatty acid having 12 or more and 18 or less carbon atoms and an amino acid is exemplified. Here, the amino acid includes aspartic acid, glutamic acid, alanine, glycine, sarcosine, proline, and hydroxyproline. Further, the acylated amino acid also includes total hydrolysates such as peptides derived from plants such as wheat and peas, silk peptides, and peptides derived from animals. The carboxyl group of the amino acid may be in a free form or in the form of a salt of K, Na, Fe, Zn, Ca, Mg, Al, Zr, Ti, or the like. For example, it is exemplified by disodium stearoyl glutamate.

Concretely, Amisoft CS-11, CS-22, MS-11, HS-11P, HS-21P, etc., which are commercially available from Ajinomoto Co., Inc., Soypon SLP, Soypon SCA, and Alanon AMP, which are commercially available from Kawaken Fine Chemicals, Co., Ltd., SEPILIFT DPHP, etc., which are commercially available from French SEPPIC Company, and Sarcosinate MN, etc., which are commercially available from Nikko Chemical Co., Ltd. can be exemplified. The acylated amino acids may be in the form of a composition with a fatty acid. Examples of the acylated lipoamino acid composition include SEPIFEEL ONE (a composition composed of four components of palmitoyl proline, palmitoyl sarcosine, palmitoyl glutamate, and palmitic acid) commercially available from SEPPIC Company.

As the fatty acid, a linear or branched saturated or unsaturated fatty acid having 12 to 22 carbon atoms is exemplified. For example, it is exemplified by a fatty acid such as lauric acid, myristic acid, palmitic acid, stearic acid, oleic acid, linoleic acid, palmitoleic acid, behenic acid, lignoceric acid, 2-ethylhexanoic acid, isotridecanoic acid, isomyristic acid, isopalmitic acid, isostearic acid, or isobehenic acid, or a metal salt of Ca, Mg, Zn, Zr, Al, Ti, or the like.

As the lecithin, natural lecithin extracted from egg yolk, soybeans, corns, rapeseed, sunflower, or the like is exemplified, and also a glyceride which is obtained by hydrogenating synthetic lecithin and is a hydrogenated lecithin having an iodine value of 15 or less and has a phosphate group is exemplified. Examples of the lecithin in the form of a salt include water-insoluble hydrogenated lecithin metal salts of Al, Mg, Ca, Zn, Zr, Ti, or the like.

The ester oil includes an ester compound having 16 or more carbon atoms in total, which can be obtained by allowing one type or two or more types of alcohols having 1 to 36 carbon atoms to react with one type or two or more types of carboxylic acids having 1 to 36 carbon atoms. Here, a compound having an acid value of 15 or more is preferred. Specific examples thereof include Salacos MIS (isostearyl sebacate), Salacos MOD (octyldodecanol azelate), Salacos 1A (octyldodecanol adipate), and Salacos HD (octyldodecanol dimerate) of Nisshin Oillio Group, Ltd.

A dextrin fatty acid ester or a fructooligosaccharide fatty acid ester is an ester composed of a dextrin or a fructooligosaccharide and a fatty acid, or a derivative thereof, and is exemplified by, for example, Rheopearl KL, Rheopearl MKL, Rheopearl TT, Rheopearl KE, Rheopearl TL, or Rheopearl ISK which is commercially available from Chiba Flour Milling Co., Ltd., or the like.

The acrylic polymer is a copolymer of one or more types of monomers composed of acrylic acid or methacrylic acid and an alkyl acrylate, and is exemplified by, for example, as an INCI name, an (acrylate/(C10-30) alkyl acrylate) crosspolymer, an (acrylate/behenes-25 methacrylate) copolymer, an (acrylate/steareth-20 methacrylate) crosspolymer, or the like.

A polyurethane polymer is a polymer having a hydrophilic group portion of a polyurethane skeleton and has a hydrophobic moiety in the molecule, and is exemplified by, for example, as an INCI name, a (PEG-240/decyltetradeceth-20/HDI) copolymer (ADEKA NOL GT-700; ADEKA Corporation), a bis-stearyl PEG/PPG-8/6 (SMDI/PEG-400) copolymer (Aqupec HU C2002; Sumitomo Seika Chemicals Company, Limited), or the like.

### [Hydrophobic Inorganic Powder (Summary)]

As described above, the hydrophobic inorganic powder can be prepared in various ways by the combination of the type of inorganic powder as the base and the type of organic surface treatment agent (single or multiple), but need only be an inorganic powder made hydrophobic by coating the surface with an organic surface treatment agent. The hydrophobic inorganic powder described in detail in the present application includes those listed below, and all of them are generally available.
Octyltriethoxysilane-treated hydrophobic pigment grade titanium oxide (trade name: ALT-TSR-10; Miyoshi Kasei, Inc.)
Octyltriethoxysilane-treated hydrophobic yellow iron oxide (trade name: ALT-YHP-10; Miyoshi Kasei, Inc.)
Octyltriethoxysilane-treated hydrophobic red iron oxide (trade name: ALT-RHP-10; Miyoshi Kasei, Inc.)
Octyltriethoxysilane-treated hydrophobic black iron oxide (trade name: ALT-BHP-10; Miyoshi Kasei, Ltd.)
Disodium stearoyl glutamate-treated hydrophobic pigment grade titanium oxide (trade name: NAI-TSR-10; Miyoshi Kasei, Inc.)
Hydrogen dimethicone-treated hydrophobic pigment grade titanium oxide (trade name: SI-TSR-10; Miyoshi Kasei, Inc.)
Hydrogen dimethicone-treated hydrophobic yellow iron oxide (trade name: SI-YHP-10; Miyoshi Kasei, Inc.)
Hydrogen dimethicone-treated hydrophobic red iron oxide (trade name: SI-RHP-10; Miyoshi Kasei, Inc.)
Hydrogen dimethicone-treated hydrophobic black iron oxide (trade name: SI-BHP-10; Miyoshi Kasei, Inc.)
Dimethylpolysiloxane and methyl hydrogen polysiloxane hybrid treated hydrophobic fine particle titanium oxide (trade name: SAS-UT-A30; Miyoshi Kasei, Inc.)
Dimethylpolysiloxane and octyltriethoxysilane hybrid treated hydrophobic fine particle zinc oxide (trade name: SALT-MZ-500; Miyoshi Kasei, Inc.)
Hydrogen dimethicone-treated hydrophobic fine particle zinc oxide (trade name: SI-fine zinc oxide; Miyoshi Kasei, Inc.)
Hydrogen dimethicone-treated talc (trade name: SI-talc JA-46R; Miyoshi Kasei, Inc.)

The hydrophobic inorganic powder itself is generally available, and therefore, the details of the hydrophobization treatment are omitted, but it can be prepared with reference to, for example, WO 2014/102862. Here, in order to further enhance the self-dispersibility in water, it is preferred to perform the hydrophobization treatment so that the particle surface becomes uniform in a state as close to the primary particle as possible during the production of the hydrophobic inorganic powder.

### [Nonionic Surfactant as Hydrophilic Coat]

The hydrophilic coat means a coat that is hydrophilic and covers the surface of the powder, and in the present application, particularly means a coat that is hydrophilic and covers the surface of the hydrophobic inorganic powder (also referred to as a hydrophilic second coat). In the present application, the hydrophobic inorganic powder covered with the hydrophilic coat is referred to as a hydrophilized inorganic powder. Here, since the hydrophilic coat is formed from a hydrophilization treatment agent (particularly, a nonionic surfactant), the composition of the hydrophilic coat can be said to be a nonionic surfactant. In the hydrophilized inorganic powder of the present application, the nonionic surfactant as the hydrophilic coat is a key component for self-dispersibility in water or an aqueous solvent.

The nonionic surfactant means a surfactant that does not ionize in water, and basically means a surfactant having a structure in which a hydrophilic moiety and a hydrophobic moiety are bonded through an ether bond or an ester bond. Note that a glycerin conjugate (glyceride) to which a structure in which a hydrophilic moiety and a hydrophobic moiety are bonded through an ether bond or an ester bond is linked by polyoxyethylene glycerin is also included in the nonionic surfactant.

### [Hydrophilic moiety]

The hydrophilic moiety means a moiety having a structure in which ethylene oxide is polymerized (that is, a polyoxyethylene structure) or a structure in which glycerin is polymerized (that is, a polyglycerin structure). Specifically, the polyoxyethylene structure is a structure that can be represented by H-(OCH₂CH₂)ₙ-, and is sometimes simply denoted by "POE". It is also referred to as polyethylene glycol and is sometimes denoted by "PEG". Further, the polyglycerin structure is a structure that can be represented by H-(OCH₂CHOHCH₂)ₙ-, and is sometimes simply denoted by "PG". In the above formula, n indicates the degree of polymerization of ethylene oxide or glycerin, and is generally also referred to as the number of moles added. For example, a polyoxyethylene structure in which the number of moles added is 10 can be represented by POE (10). The number of moles added is an average value or a peak value. For example, in POE (10), POE (9), POE (11), or the like can be mixed. Further, the polyglycerin structure in which the number of moles added is 5 can be represented by PG (5).

### [Branched Carbon Chain Moiety (Hydrophobic Moiety)]

A carbon chain moiety means a moiety derived from a higher alcohol or a higher fatty acid, and can also be referred to as a hydrophobic moiety. The carbon chain moiety derived from a higher alcohol is bonded to the hydrophilic moiety through an ether bond, and therefore has a structure that can be represented by -O(CH₂)ₘH. Further, the carbon chain moiety derived from a higher fatty acid is bonded to the hydrophilic moiety through an ester bond, and therefore has a structure that can be represented by -OCO(CH₂)ₘ₋₁H. Note that m in the above formulae corresponds to the number of carbon atoms in the carbon chain moiety. The number of carbon atoms is also an average value or a peak value. The number of carbon atoms in the carbon chain moiety can also be represented by, for example, (C₁₈).

The branched structure means a structure of a branched carbon chain, that is, a non-linear carbon chain moiety. The hydrophobic moiety of the nonionic surfactant which is the hydrophilic coat of the present invention is a branched higher alcohol or a branched higher fatty acid. In the present application, the branched structure is classified into a monomethyl type, a dimethyl type, and a multi-branched type, but the branched type does not matter as long as it is a branched structure sufficient to impart self-dispersibility to the hydrophobic inorganic powder.

### [Nonionic Surfactants Used in Examples and Comparative Examples]

As described above, nonionic surfactants can be classified according to the structures thereof, but the types of nonionic surfactants described in detail in the present application are summarized as follows.

| Type No. | Hydrophobic moiety | Hydrophilic moiety | Bonding mode | Remarks |
|---|---|---|---|---|
| Type 1 | Branched higher alcohol | POE | Monoether | Example |
| Type 2 | Branched higher fatty acid | POE | Monoester | |
| Type 3 | Branched higher fatty acid | POE glycerin ether | Ester | |
| Type 4 | Branched higher fatty acid | POE | Monoester | |
| Type 5 | Linear higher alcohol | POE | Monoether | Comparative Example |
| Type 6 | Linear higher fatty acid | POE | Monoester | |
| Type 7 | Linear higher fatty acid | POE glycerin ether | Ester | |
| Type 8 | Linear higher fatty acid | POE | Monoester | |

### [Nonionic Surfactant of Type 1]

Type 1 is a nonionic surfactant in which POE and a branched higher alcohol are bonded through a monoether bond, and is also referred to as a polyoxyethylene monobranched higher alkyl ether. The number of moles added of POE is preferably 5 to 20. The number of carbon atoms in the branched higher alcohol is preferably 12 to 20. The number of moles added of POE and the number of carbon atoms of the branched higher alcohol are the same for the other types described below. Examples of the branched higher alcohol include isododecanol (C₁₂), isomyristyl alcohol (C₁₄), isocetyl alcohol (C₁₆), isostearyl alcohol (C₁₈), and isoeicosanol (C₂₀), and the like. Examples of the nonionic surfactant of Type 1 that is generally available include Nonion IS-205 (polyoxyethylene-5 isostearyl ether: HLB 9.0), Nonion IS-210 (polyoxyethylene-10 isostearyl ether: HLB 12.4), Nonion IS-215 (polyoxyethylene-15 isostearyl ether: HLB 14.2), Nonion IS-220 (polyoxyethylene-20 isostearyl ether: HLB 15.3), and Nonion OD-220 (polyoxyethylene-20 octyldodecyl ether: HLB 14.9) of NOF Corporation, and EMALEX 1605 (polyoxyethylene-5 hexyldecyl ether: HLB 9.5), EMALEX 1610 (polyoxyethylene-10 hexyldecyl ether: HLB 12.9), EMALEX 1805 (polyoxyethylene-5 isostearyl ether: HLB 9.0), EMALEX 1810 (polyoxyethylene-10 isostearyl ether: HLB 12.4), and EMALEX 1815 (polyoxyethylene-15 isostearyl ether: HLB 14.2) of Nihon Emulsion Co., Ltd, and the like.

### [Nonionic Surfactant of Type 2]

Type 2 is a nonionic surfactant in which POE and a branched higher fatty acid are bonded through a monoester bond, and is also referred to as a polyoxyethylene branched higher fatty acid monoester. Examples of an iso-type fatty acid include isododecanoic acid (C₁₂), isomyristic acid (C₁₄), isopalmitic acid (C₁₆), isostearic acid (C₁₈), and isoeicosanoic acid (C₂₀). Examples of the nonionic surfactant of Type 2 that is generally available include Nonion IS-4 (polyoxyethylene-8 isostearate: HLB 11.6), Nonion IS-6 (polyoxyethylene-12 isostearate: HLB 13.7) of NOF Corporation, and the like.

### [Nonionic Surfactant of Type 3]

Type 3 is a nonionic surfactant in which an etherified product of POE and glycerin and a branched higher fatty acid are bonded through an ester bond, and is also referred to as a polyoxyethylene glyceryl branched higher fatty acid ester. There are a monoester type, a diester type, and a triester type, but the ester type does not matter as long as it has a structure sufficient to impart self-dispersibility to the hydrophobic inorganic powder. Examples of the nonionic surfactant of Type 3 that is generally available include Uniox GT-20IS (polyoxyethylene-20 glyceryl triisostearate: HLB 10.4), Uniox GT-30IS (polyoxyethylene-30 glyceryl triisostearate: HLB 12.3), Uniox GM-8IS (polyoxyethylene-8 glyceryl isostearate: HLB 12.0) of NOF Corporation, and the like.

### [Nonionic Surfactant of Type 4]

Type 4 is a nonionic surfactant in which PG and a branched higher fatty acid are bonded through a monoester bond, and is also referred to as a polyglycerin branched higher fatty acid ester. The degree of polymerization of PG is the same as that of POE. Examples of the nonionic surfactant of Type 4 that is generally available include Decaglyn 1-ISV (polyglyceryl-10 monoisostearate: HLB 15.5) of Nikko Chemicals Co., Ltd, and the like.

### [Nonionic Surfactant of Type 5]

Type 5 is a nonionic surfactant in which POE and a linear higher alcohol are bonded through a monoether bond. Here, a case where a linear higher alcohol including a double bond is used is also included in Type 5. Examples of the nonionic surfactant of Type 5 include Nonion K-209 (polyoxyethylene (9) lauryl ether: HLB 13.6), Nonion S-207 (polyoxyethylene (7) stearyl ether: HLB 10.7), Nonion E-205S (polyoxyethylene (5) oleyl ether: HLB 9.0) of NOF Corporation, and the like.

### [Nonionic Surfactant of Type 6]

Type 6 is a nonionic surfactant in which POE and a linear higher fatty acid are bonded through a monoester bond. Examples of the nonionic surfactant of Type 6 include RHEODOL TW-O120V (polyoxyethylene (20) monooleate: HLB 15.0) of Kao Corporation, and the like.

### [Nonionic Surfactant of Type 7]

Type 7 is a nonionic surfactant in which an etherified product of POE and glycerin and a linear higher fatty acid are bonded through an ester bond. Examples thereof include EMALEX GWS-320 (polyoxyethylene (20) glyceryl tristearate: HLB 11.6) of Nihon Emulsion Co., Ltd, and the like.

### [Nonionic Surfactant of Type 8]

Type 8 is a nonionic surfactant in which PG and a linear higher fatty acid are bonded through a monoester bond, and is also referred to as a polyglycerin linear higher fatty acid ester. The degree of polymerization of PG is the same as that of POE. Examples of the nonionic surfactant of Type 8 that is generally available include Decaglyn 1-SV (polyglyceryl-10 monostearate: HLB 15.5) of Nikko Chemicals Co., Ltd, and the like.

### [Modifications of Nonionic Surfactant]

As described above, the nonionic surfactant basically has a structure in which a hydrophilic moiety and a hydrophobic moiety are bonded through an ether bond or an ester bond, and nonionic surfactants other than the above-mentioned Types can also be included in the present application. Examples of the types other the above-mentioned Types include a polyglycerin branched higher alcohol ether (that is, PG version of Type 1), a polyglycerin branched higher fatty acid ester (that is, PG version of Type 2) , and the like. Here, the properties of the polyglycerin branched higher alkyl ether can be predicted by those skilled in the art from the properties of Type 1, and it is included in the scope of the present application.

In addition, another structure may intervene between the hydrophilic moiety and the hydrophobic moiety of the nonionic surfactant. Examples of the another structure include sorbitan, erythritol, and sucrose. Specifically, it has a structure called a polyoxyethylene sorbitan branched higher fatty acid ester, a polyoxyethylene erythritol branched higher fatty acid ester, a polyoxyethylene sucrose branched higher fatty acid ester, a polyglycerin branched higher fatty acid ester, or the like, and is exemplified by, for example, Nonion IST-221 (polyoxyethylene-20 sorbitan isostearate: HLB 15.7) of NOF Corporation.

Further, it may be a polyoxyethylene branched higher fatty acid hydrogenated castor oil ester (for example, Uniox HC-20MIS or Uniox HC-40MIS of NOF Corporation) or the like.

### [HLB]

The HLB (Hydrophilic-Lipophilic Balance) is a value indicating the degree of affinity of a surfactant for water and oil. The HLB is calculated by the following formula in the present application. HLB = (Molecular weight of hydrophilic moiety (POE or PG) in surfactant/Molecular weight of surfactant) × 20

### [Hydrophilized Inorganic Powder]

The hydrophilized inorganic powder means a powder including an inorganic powder as a base material, a hydrophobic coat that covers the surface of the inorganic powder, and a hydrophilic coat that covers the hydrophobic coat. That is, when regarding the inorganic powder as a starting material, first, the surface of the inorganic powder is coated with an organic surface treatment agent(s) to form a hydrophobic inorganic powder, and then, the resulting powder is further covered with a nonionic surfactant(s) (that is, a hydrophilization treatment) to form a hydrophilized inorganic powder, so that the resulting powder has a double coat of the hydrophobic coat formed from the organic surface treatment agent(s) and the hydrophilic coat formed from the nonionic surfactant(s).

The hydrophilization treatment method is not particularly limited, and the preparation can be carried out by mixing in a state where the nonionic surfactant(s) and the hydrophobic inorganic powder are in contact with each other. The mixing method is also not particularly limited, and a mixing machine capable of uniformly performing the treatment may be adopted. For example, a Henschel mixer, a ribbon blender, a kneader, an extruder, a disper mixer, a homomixer, a bead mill, and the like are exemplified. After mixing, drying is performed with a hot air dryer, a spray dryer, a flash dryer, a conical dryer, or the like, whereby the powder can be obtained.

The blending ratio of the nonionic surfactant (A) and the hydrophobic inorganic powder (B) is (A)/(B) = 0.1/99.9 to 20.0/80.0 (wt%). The blending ratio is preferably 0.1/99.9 to 15.0/85.0 (wt%), and more preferably 0.1/99.9 to 10.0/90.0 (wt%). The blending amount of the surfactant is preferably as small as possible from the viewpoint of irritation to the skin.

### [Water and Aqueous Solvent]

The hydrophilized inorganic powder of the present application has self-dispersibility in water. Water as used herein refers to ion exchanged water, distilled water, or the like. As water to be blended in a cosmetic, aseptic or sterilized water is used. The aqueous solvent of the present application refers to a liquid containing a water-soluble alcohol as another component. Examples thereof include alcohols such as ethanol, a polyhydric alcohol, propylene glycol, dipropylene glycol, 1,3-butylene glycol, polyethylene glycol, glycerin, diglycerin, polyglycerin, hexylglycerin, cyclohexylglycerin, trimethylolpropane, xylitol, erythritol, trehalose, and sorbitol. The blending ratio of water and an alcohol is water/alcohol = 100/0 to 50/50 (wt%), but the blending ratio of the alcohol is preferably as low as possible from the viewpoint of self-dispersibility of the hydrophilized inorganic powder.

Further, as an intermediate raw material in the production of a cosmetic, an aqueous dispersion composition containing the hydrophilized inorganic powder at a high concentration can be considered. The aqueous dispersion composition is a composition in which the hydrophilized inorganic powder is dispersed as a main component in water, and can be in the form of a liquid that flows or in the form of particles. The use of a disperser in the step of producing the dispersion composition has advantages in terms of usability that the dispersion state of the hydrophilized inorganic powder can be adjusted, the powder particles can be prevented from scattering when blended in a cosmetic, and the like.

In the aqueous dispersion composition, as another component(s), a surfactant, a moisturizer, a UV absorber, a preservative, an antioxidant, a coat forming agent, a thickener, a dye, a pigment, various chemicals, a fragrance, or the like can be appropriately blended.

As the surfactant(s), a nonionic surfactant, particularly, polyoxyethylene (10) isostearyl ether can be contained, but it is clearly distinguished from one used at the time of preparing the hydrophilized inorganic powder (one for the use of the present invention).

The thickener can be added for the purpose of stably dispersing the hydrophilized inorganic powder in the aqueous dispersion composition over a long period of time, in other words, ensuring the storage stability. That is, the hydrophilized inorganic powder floats or precipitates or liquid separation can occur depending on the difference in specific gravity between the hydrophilized inorganic powder and water or the aqueous solvent depending on the type of the inorganic powder or the like. Here, if the thickener is added to water or the aqueous solvent, the floating or precipitation of the hydrophilized inorganic powder can be suppressed.

Examples of the thickener include sodium hyaluronate, cationized sodium hyaluronate, a polymer and a copolymer having acryloyldimethyltaurine or a salt thereof as a constituent unit, and polyvinylpyrrolidone. Specific examples thereof include (sodium acrylate/ acryloyldimethyltaurate/dimethylacrylamide) crosspolymer (trade name: SEPINOV P88; Seiwa Kasei Co., Ltd.), polyacrylate crosspolymer-6 (trade name: SEPIMAX ZEN; Seiwa Kasei Co., Ltd.), (hydroxyethyl acrylate/sodium acryloyldimethyltaurate) copolymer (trade name: SEPINOV EMT 10; Seiwa Kasei Co., Ltd.), polyvinylpyrrolidone (trade name: Luviskol K17, Luviskol K30, Luviskol K90; BASF Japan Ltd.), a (PEG-240/decyltetradeceth-20/HDI) copolymer/potassium laurate/BG/water mixture (ADEKA NOL GT-730; ADEKA Corporation), a polyurethane-59/BG/water mixture (ADEKA NOL GT-930; ADEKA Corporation), Trideses-6 (Avalure Flex-6 CC Polymer; Lubrizol Corporation), Xanthan gum (KELTROL CG-T; Sansho Co., Ltd.), gellan gum (Kelcogel, Kelcogel HM; DSP Gokyo Food & Chemical Co. Ltd.), silicic acid (Na/Mg) (trade name: OVEIL ER (Osaka Gas Chemical Co., Ltd.)), bentonite (trade name: OVEIL BR (Osaka Gas Chemical Co., Ltd.)), and the like.

### [Cosmetic]

The cosmetic includes a makeup cosmetic, a skin care cosmetic, a hair cosmetic, and the like. Examples of the makeup cosmetic include a makeup base, a white powder foundation (water-based, oil-based), a powder foundation, a liquid foundation, a W/O-type emulsion foundation, an oily foundation, an oily solid foundation, a stick foundation, a pressed powder, a face powder, a white powder, a lipstick, a lipstick overcoat, a lip gloss, a concealer, a cheek color, an eye shadow (water-based, oil-based), an eyebrow, an eyeliner, a mascara, an aqueous nail enamel, an oily nail enamel, an emulsion nail enamel, an enamel top coat, and an enamel base coat. Examples of the skin care cosmetic include an emollient cream, a cold cream, a whitening cream, a milky lotion, a toner lotion, a beauty essence serum, a facial pack, a carmine lotion, a liquid face wash, a face wash foam, a face wash cream, a face wash powder, a makeup cleansing, a body gloss, a sunscreen or suntan cream or lotion, a water-based suncut lotion, an O/W-type sunscreen cosmetic, and the like. Examples of the hair cosmetic include a hair gloss, a hair cream, a hair shampoo, a hair rinse, a hair color, a hair brushing agent, a hair treatment, and the like. Examples of an antiperspirant include cream, lotion, powder, spray-type deodorant products, and the like. In addition, a milky lotion, a soap, a bathing agent, a perfume, and the like are also included in the cosmetic in the present application.

### EXAMPLES

Hereinafter, the present invention will be described in detail with reference to preferred Examples and Comparative Examples corresponding to the Examples. The present invention is not limited to the following Examples, and modification, change, application (including partial one) and combination thereof can be made without deviating from the technical meaning of the present invention found from the following Examples.

### [Evaluation of Hydrophilicity and Self-Dispersibility of Hydrophilized Inorganic Powder]

First, hydrophilized inorganic powders shown in the following Examples 1 to 17 and Comparative Examples 1 to 7 were prepared, and the hydrophilicity and self-dispersibility of each powder were evaluated.

### (Example 1)

102 g of polyoxyethylene (10) isostearyl ether (Nonion IS-210) was added to 1.5 kg of ion exchanged water and dissolved in a white turbid state at 60°C. The resulting white turbid solution was added to 5 kg of octyltriethoxysilane-treated hydrophobic pigment grade titanium oxide (trade name: ALT-TSR-10; Miyoshi Kasei, Inc.), kneaded with a kneader mixer for 30 minutes, and then stirred under vacuum heating to distill off ion exchanged water, whereby a powder of Example 1 was obtained.

### (Example 2)

208 g of polyoxyethylene (10) isostearyl ether (Nonion IS-210) was dissolved in 2 kg of an ion exchanged water/isopropyl alcohol (IPA) = 8/2 (wt%) solution. The resulting solution was added to 5 kg of octyltriethoxysilane-treated hydrophobic yellow iron oxide (trade name: ALT-YHP-10; Miyoshi Kasei, Inc.), and a treatment was performed in the same manner as in Example 1, whereby a powder of Example 2 was obtained.

### (Example 3)

208 g of polyoxyethylene (10) isostearyl ether (Nonion IS-210) was dissolved in 2 kg of an ion exchanged water/isopropyl alcohol (IPA) = 8/2 (wt%) solution. The resulting solution was added to 5 kg of octyltriethoxysilane-treated hydrophobic red iron oxide (trade name: ALT-RHP-10; Miyoshi Kasei, Inc.), and a treatment was performed in the same manner as in Example 1, whereby a powder of Example 3 was obtained.

### (Example 4)

155 g of polyoxyethylene (10) isostearyl ether (Nonion IS-210) was dissolved in 1.2 kg of an ion exchanged water/isopropyl alcohol (IPA) = 8/2 (wt%) solution. The resulting solution was added to 5 kg of octyltriethoxysilane-treated hydrophobic black iron oxide (trade name: ALT-BHP-10; Miyoshi Kasei, Inc.), and a treatment was performed in the same manner as in Example 1, whereby a powder of Example 4 was obtained.

### (Example 5)

102 g of polyoxyethylene (10) isostearyl ether (Nonion IS-210) was added to 200 g of ion exchanged water and dissolved in a white turbid state. 5 kg of disodium stearoyl glutamate-treated hydrophobic pigment grade titanium oxide (trade name: NAI-TSR-10; Miyoshi Kasei, Inc.) and the resulting white turbid solution were added to a heater Henschel and stirred for 30 minutes. Heated steam was allowed to pass through the jacket of the heater Henschel and ion exchanged water was distilled off by stirring with heating. The resultant was pulverized with an atomizer, whereby a powder of Example 5 was obtained.

### (Example 6)

102 g of polyoxyethylene (10) isododecyl ether (Nonion ISK-210) was dissolved in a white turbid state in 200 g of an ion exchanged water/isopropyl alcohol (IPA) = 8/2 (wt%) solution. 5 kg of hydrogen dimethicone-treated hydrophobic pigment grade titanium oxide (trade name: SI-TSR-10; Miyoshi Kasei, Inc.) was added thereto, and a treatment was performed in the same manner as in Example 5, whereby a powder of Example 6 was obtained.

### (Example 7)

208 g of polyoxyethylene (10) isostearyl ether (Nonion IS-210) was dissolved in 250 g of an ion exchanged water/isopropyl alcohol (IPA) = 8/2 (wt%) solution. The resulting solution was added to 5 kg of hydrogen dimethicone-treated hydrophobic yellow iron oxide (trade name: SI-YHP-10; Miyoshi Kasei, Inc.), and a treatment was performed in the same manner as in Example 5, whereby a powder of Example 7 was obtained.

### (Example 8)

208 g of polyoxyethylene (10) isostearyl ether (Nonion IS-210) was dissolved in 250 g of an ion exchanged water/isopropyl alcohol (IPA) = 8/2 (wt%) solution. The resulting solution was added to 5 kg of hydrogen dimethicone-treated hydrophobic red iron oxide (trade name: SI-RHP-10; Miyoshi Kasei, Inc.), and a treatment was performed in the same manner as in Example 5, whereby a powder of Example 8 was obtained.

### (Example 9)

102 g of polyoxyethylene (10) isostearyl ether (Nonion IS-210) was dissolved in 200 g of an ion exchanged water/isopropyl alcohol (IPA) = 8/2 (wt%) solution. The resulting solution was added to 5 kg of hydrogen dimethicone-treated hydrophobic black iron oxide (trade name: SI-BHP-10; Miyoshi Kasei, Inc.), and a treatment was performed in the same manner as in Example 5, whereby a powder of Example 9 was obtained.

### (Example 10)

To 3 kg of ion exchanged water, 158 g of polyoxyethylene (12) isostearate (Nonion IS-6) was added and dissolved in a white turbid state at 60°C. A disper mixer was placed in the resulting solution, and while stirring the solution, 3 kg of dimethylpolysiloxane and methyl hydrogen polysiloxane hybrid treated hydrophobic fine particle titanium oxide (trade name: SAS-UT-A30; Miyoshi Kasei, Inc.) was gradually added thereto, whereby an aqueous dispersion was formed. The resulting aqueous dispersion was spray-dried with a spray dryer with two-fluid nozzles, whereby a powder of Example 10 was obtained.

### (Example 11)

To 3 kg of ion exchanged water, 125 g of polyoxyethylene (20) glyceryl triisostearate (Uniox GT-20IS) was added and dissolved at 60°C. A disper mixer was placed in the resulting solution, and while stirring the solution, 5 kg of dimethylpolysiloxane and octyltriethoxysilane hybrid treated hydrophobic fine particle zinc oxide (trade name: SALT-MZ-500; Miyoshi Kasei, Inc.) was gradually added thereto, whereby an aqueous dispersion was formed. The resulting aqueous dispersion was spray-dried with a spray dryer with two-fluid nozzles, whereby a powder of Example 11 was obtained.

### (Example 12)

A powder of Example 12 was obtained by performing a procedure in the same manner as in Example 1 except that the polyoxyethylene (10) isostearyl ether in Example 1 was changed to polyglyceryl (10) monoisostearate (Decaglyn 1-ISV).

### (Example 13)

A powder of Example 13 was obtained by performing a procedure in the same manner as in Example 1 except that the polyoxyethylene (10) isostearyl ether in Example 1 was changed to polyoxyethylene (20) octyldodecyl ether (EMALEX OD-20).

### (Example 14)

A powder of Example 14 was obtained by performing a procedure in the same manner as in Example 1 except that the polyoxyethylene (10) isostearyl ether in Example 1 was changed to polyoxyethylene (8) glyceryl isostearate (Uniox GM-8IS).

### (Example 15)

A powder of Example 15 was obtained by performing a procedure in the same manner as in Example 1 except that the polyoxyethylene (10) isostearyl ether in Example 1 was changed to polyoxyethylene (8) glyceryl isostearate (Uniox GM-8IS).

### (Example 16)

102 g of polyoxyethylene (5) hexyldecyl ether (EMALEX 1605) was added to 1.5 kg of ion exchanged water and dissolved at 60°C. The resulting white turbid solution was added to 5 kg of hydrogen dimethicone-treated hydrophobic fine particle zinc oxide (trade name: SI-fine zinc oxide; Miyoshi Kasei, Inc.), and kneaded with a kneader mixer for 30 minutes. The resulting kneaded material was dried and pulverized with a flash dryer, whereby a powder of Example 16 was obtained.

### (Example 17)

A powder of Example 17 was obtained by performing a procedure in the same manner as in Example 5 except that the hydrophobic pigment grade titanium oxide in Example 5 was changed to hydrogen dimethicone-treated talc (trade name: SI-talc JA-46R; Miyoshi Kasei, Inc.).

### (Comparative Example 1)

A powder of Comparative Example 1 was obtained by performing a procedure in the same manner as in Example 1 except that the polyoxyethylene (10) isostearyl ether in Example 1 was changed to polyoxyethylene (10) stearyl ether (trade name: EMALEX 610 (HLB 12.4); Nihon Emulsion Co., Ltd.).

### (Comparative Example 2)

A powder of Comparative Example 2 was obtained by performing a procedure in the same manner as in Example 6 except that the polyoxyethylene (10) isododecyl ether in Example 6 was changed to polyoxyethylene (10) lauryl ether (trade name: Nonion K-210 (HLB 14.1); NOF Corporation)).

### (Comparative Example 3)

A powder of Comparative Example 3 was obtained by performing a procedure in the same manner as in Example 7 except that the polyoxyethylene (10) isostearyl ether in Example 7 was changed to polyglyceryl (10) monostearate (trade name: Decaglyn 1-SV (HLB *15.5)).*

### (Comparative Example 4)

A powder of Comparative Example 4 was obtained by performing a procedure in the same manner as in Example 8 except that the polyoxyethylene (10) isostearyl ether in Example 8 was changed to polyoxyethylene (5) oleyl ether (trade name: Nonion E-205S (HLB 9.0); NOF Corporation).

### (Comparative Example 5)

A powder of Comparative Example 5 was obtained by performing a procedure in the same manner as in Example 9 except that the polyoxyethylene (10) isostearyl ether in Example 9 was changed to polyoxyethylene (20) monooleate (trade name: RHEODOL TW-O120V (HLB 15.0); Kao Corporation).

### (Comparative Example 6)

A powder of Comparative Example 6 was obtained by performing a procedure in the same manner as in Example 10 except that the polyoxyethylene (12) isostearate in Example 10 was changed to polyoxyethylene (6) isodecyl ether (trade name: Nonion ID-203 (HLB 9.1); NOF Corporation).

### (Comparative Example 7)

A powder of Comparative Example 7 was obtained by performing a procedure in the same manner as in Example 11 except that the polyoxyethylene (20) glyceryl triisostearate in Example 11 was changed to polyoxyethylene (20) glyceryl tristearate (trade name: EMALEX GWS-320 (HLB 11.6); Nihon Emulsion Co., Ltd.).

### (Comparative Example 8)

The inorganic powders as the bases of the powders of the respective Examples and Comparative Examples were prepared as they were for comparative evaluation. Unlike the powders of the respective Examples and Comparative Examples, these inorganic powders (untreated) do not have both a hydrophobic coat and a hydrophilic coat, and the inorganic powders themselves are exposed and hydrophilic.

When the nonionic surfactants used in the above Examples and Comparative Examples are applied to the above-mentioned classification, the surfactants are classified as follows.

| Type No. | Nonionic surfactant | HLB | Remarks |
|---|---|---|---|
| Type 1 | POE (20) octyldodecyl ether | 14.9 | Example 13 |
| | POE (10) isostearyl ether | 12.4 | Examples 1 to 5, 7 to 9, and 17 |
| | POE (5) hexyldecyl ether | 9.5 | Example 16 |
| | POE (10) isodecyl ether | 14.1 | Example 6 |
| | POE (6) isodecyl ether | 9.1 | Comparative Example 6 |
| Type 2 | POE (12) isostearate | 13.7 | Example10 |
| Type 3 | POE (8) glyceryl monoisostearate | 12.0 | Examples 14 and 15 |
| | POE (20) glyceryl triisostearate | 10.4 | Example 11 |
| Type 4 | PG (10) isostearate | 15.5 | Example 12 |
| Type 5 | POE (10) stearyl ether | 12.4 | Comparative Example 1 |
| | POE (10) lauryl ether | 14.1 | Comparative Example 2 |
| | POE (5) oleyl ether | 9.0 | Comparative Example 4 |
| Type 6 | POE (20) monooleate | 15.3 | Comparative Example 5 |
| Type 7 | POE (20) glyceryl tristearate | 10.4 | Comparative Example 7 |
| Type 8 | PG (10) stearate | 15.5 | Comparative Example 3 |

### (Evaluation Method for Hydrophilicity and Self-Dispersibility)

### TEST 1

**100** cc of ion exchanged water was placed in a 200 cc glass vial, and 0.3 g of a powder was taken out with a spatula and dropped from a height of 5 cm above the surface of water, and a state where the powder particles fall into water was observed according to the following evaluation criteria. After dropping the powder on the surface of water, a property that the powder particles spontaneously diffuse and disperse in water (that is, self-dispersibility) without performing any physical stirring operation was observed.

### TEST 2

Evaluation was performed in the same manner as in Test 1 except that the ion exchanged water in Test 1 was changed to an ion exchanged water/butylene glycol (BG) = 6/4 (wt%) solution.

### TEST 3

Evaluation was performed in the same manner as in Test 1 except that the ion exchanged water in Test 1 was changed to an ion exchanged water/glycerin (G) = 6/4 (wt%) solution.

### (Evaluation Criteria for Hydrophilicity and Self-Dispersibility)

A: The powder particles self-dispersed (diffused) in the aqueous layer, and after 60 seconds, the powder particles diffused in the entire aqueous layer and caused turbidity.
B: The powder particles entered the aqueous layer, but after 60 seconds, a small part of the aqueous layer became turbid with the powder particles.
C: The powder particles did not disperse in the aqueous layer, but precipitated or floated.

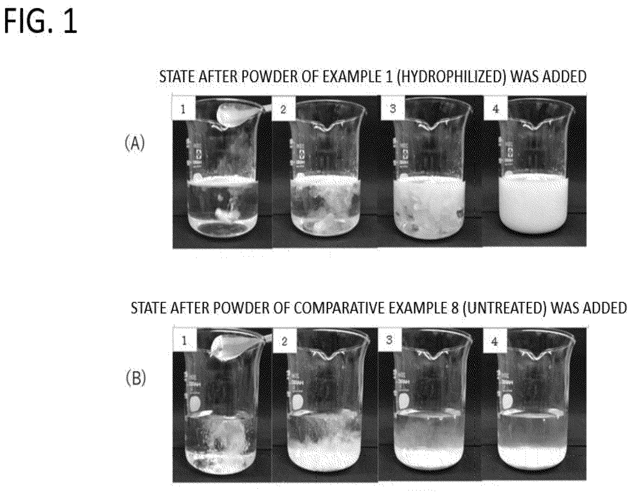

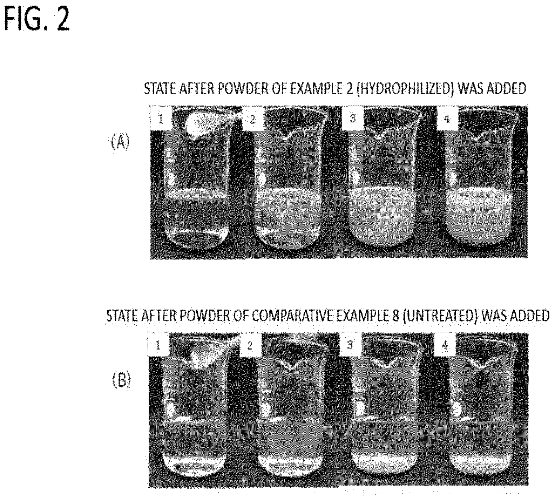

FIGS. 1 and 2 show evaluation examples of hydrophilicity and self-dispersibility, and show changes in state with the passage of time from (1) immediately after each powder was added to ion exchanged water to (4) 60 seconds later. FIG. 1(A) shows changes in state of the powder of Example 1 (the powder obtained by performing the hydrophilization treatment of octyltriethoxysilane-treated hydrophobic pigment grade titanium oxide with polyoxyethylene (10) isostearyl ether), which is an example of the evaluation criterion: A. FIG. 1(B) shows changes in state of the powder of Comparative Example 8 (octyltriethoxysilane-treated hydrophobic pigment grade titanium oxide itself used in Example 1), which is an example of the evaluation criterion: C. FIG. 2(A) shows changes in state of the powder of Example 2 (the powder obtained by performing the hydrophilization treatment of octyltriethoxysilane-treated hydrophobic yellow iron oxide with polyoxyethylene (10) isostearyl ether), which is an example of the evaluation criterion: A. FIG. 2(B) shows changes in state of the powder of Comparative Example 8 (octyltriethoxysilane-treated hydrophobic yellow iron oxide itself used in Example 2), which is an example of the evaluation criterion: C.

### (Evaluation Results of Hydrophilicity and Self-Dispersibility)

**[Table 1]**

| | Test 1 : ion exchanged water | | Test 2: ion exchanged water/BG | | Test 3: ion exchanged water/G | |
|---|---|---|---|---|---|---|
| | Hydrophilizatio n treatment | Untreated (Comparativ e Example 8) | Hydrophilizatio n treatment | Untreated (Comparativ e Example 8) | Hydrophilizatio n treatment | Untreated (Comparativ e Example 8) |
| Example 1 | A | C | A | C | A | C |
| Example 2 | A | C | A | C | A | C |
| Example 3 | A | C | A | C | A | C |
| Example 4 | A | C | A | C | A | C |
| Example 5 | A | C | A | C | A | C |
| Example 6 | A | C | A | C | A | C |
| Example 7 | A | C | A | C | A | C |
| Example 8 | A | C | A | C | A | C |
| Example 9 | A | C | A | C | A | C |
| Example 10 | A | C | A | C | A | C |
| Example 11 | A | C | A | C | A | C |
| Example 12 | A | C | A | C | A | C |
| Example 13 | A | C | A | C | A | C |
| Example 14 | A | C | A | C | A | C |
| Example 15 | A | C | A | C | A | C |
| Example 16 | A | C | A | C | A | C |
| Example 17 | A | C | A | C | A | C |
| Comparativ e Example 1 | B | - | C | - | C | - |
| Comparativ e Example 2 | B | - | B | - | C | - |
| Comparativ e Example 3 | C | - | C | - | C | - |
| Comparativ e Example 4 | B | - | B | - | C | - |
| Comparativ e Example 5 | C | - | C | - | C | - |
| Comparativ e Example 6 | B | - | B | - | C | - |
| Comparativ e Example 7 | B | - | B | - | C | - |

### (Discussion of Evaluation Results 1)

From the evaluation results of Example 1 and Comparative Example 8, and the like, it was found that the hydrophilicity and self-dispersibility are clearly improved by subjecting the inorganic powder as the base body to both the hydrophobization treatment and the hydrophilization treatment. In particular, as shown in FIGS. 1 and 2, it was found that the powders of Examples 1 to 17 have remarkable usefulness in the property of naturally diffusing and dispersing in water without performing any physical stirring operation (that is, self-dispersibility) after being dropped into ion exchanged water or the like. More specifically, it can be said that the powders of Examples 1 to 17 spontaneously disperse and uniformly mix within several tens of seconds (within at most 60 seconds) after being dropped into an aqueous solvent.

Further, in Example 1, the blending amount of the surfactant with respect to the hydrophobic inorganic powder is 102 g/5 kg = 0.0204, and it was found that even if the blending amount is about 2 wt%, good hydrophilicity and self-dispersibility are achieved.

### (Discussion of Evaluation Results 2)

From the evaluation results of Example 1 and Comparative Example 1, it was found that when polyoxyethylene (10) isostearyl ether is used as the surfactant (Example 1: A), better hydrophilicity and self-dispersibility are achieved as compared with the case where polyoxyethylene (10) stearyl ether is used as the surfactant (Comparative Example 1: B). A different point between Example 1 and Comparative Example 1 resides in the structure of the carbon chain moiety of the surfactant (that is, Example 1: branched structure, Comparative Example 1: linear structure), and therefore, it was found that the surfactant in which the carbon chain moiety has a branched structure (that is, a nonionic surfactant of Type 1) is useful from the viewpoint of hydrophilicity and self-dispersibility. The same can be said said from the evaluation results of Example 6 and Comparative Example 2 (that is, the number of carbon atoms in the carbon chain moiety is C12 in both cases).

However, as in Comparative Example 6, when the number of moles added of polyoxyethylene is 6 and the length of the carbon chain moiety is C10, the surfactant in which the carbon chain moiety has a branched structure is poorer than in Example 1 or the like in terms of hydrophilicity and self-dispersibility. Here, the surfactant in which the number of carbon atoms in the carbon chain moiety is the smallest in Examples 1 to 17 is polyoxyethylene (10) isododecyl ether used in Example 6 with a chain length of C12, and the surfactant in which the number of carbon atoms in the carbon chain moiety is the largest in Examples 1 to 17 is polyoxyethylene (20) octyldodecyl ether used in Example 13 with a chain length of C20. Therefore, the surfactant useful from the viewpoint of hydrophilicity and self-dispersibility can be selected from the perspective that the number of carbon atoms in the carbon chain moiety is C12 to C20.

Further, the surfactant having the lowest HLB in Examples 1 to 17 is polyoxyethylene (5) hexyldecyl ether used in Example 16 with an HLB of 9.5, and the surfactant having the highest HLB in Examples 1 to 17 is polyglyceryl (10) monoisostearate used in Example 12 with an HLB of 15.5. Therefore, the surfactant useful from the viewpoint of hydrophilicity and self-dispersibility can be selected from the perspective that the HLB is 9.5 to 15.5.

### (Discussion of Evaluation Results 3)

From the evaluation results of Example 11 and Comparative Example 7, it can be said that the usefulness of the surfactant in which the carbon chain moiety has a branched structure does not depend on whether or not it is a glycerin conjugate.

### (Discussion of Evaluation Results 4)

From the evaluation results of Example 12 and Comparative Example 5, it can be said that the usefulness of the surfactant in which the carbon chain moiety has a branched structure does not depend on the bond type (that is, an ether bond or an ester bond) between the hydrophilic moiety and the carbon chain moiety.

### (Discussion of Evaluation Results 5)

From the evaluation results of Examples 1 to 4 and the like, it can be said that good hydrophilicity and self-dispersibility are achieved independently of the type of inorganic powder as the base material.

### (Discussion of Evaluation Results 6)

From the evaluation results of Examples 1, 5, 6, and the like, it can be said that good hydrophilicity and self-dispersibility are achieved independently of the composition of the hydrophobic coat. At least it can be said that the composition of the hydrophobic coat can be selected from octyltriethoxysilane, disodium stearoyl glutamate, hydrogen dimethicone, dimethylpolysiloxane, and methyl hydrogen polysiloxane.

### (Summary of Discussions of Evaluation Results)

From the above discussions, the composition of the hydrophilic coat that can achieve good hydrophilicity and self-dispersibility is a nonionic surfactant in which the carbon chain moiety has a branched structure and has 12 to 20 carbon atoms. In particular, when any one of a condition that the surfactant is a nonionic surfactant of a monoether type being Type 1 or a condition that the HLB is 9.5 to 15.5 is met, particularly good hydrophilicity and self-dispersibility can be achieved.

### [Evaluation of Sense of Use, Cosmetic Effect, and Cosmetic Durability of O/W-Type Emulsion Foundation 1]

O/W-type emulsion foundations having compositions shown in the following Example 18 and Comparative Example 9 were prepared, and the sense of use, cosmetic effect, and cosmetic durability of each O/W-type emulsion foundation were evaluated.

### (Preparation Method for O/W-Type Emulsion Foundation)

A: The oil layer components were well dispersed and mixed.
B: The aqueous layer components were well dispersed and mixed.
C: After A was added to B, the resultant was emulsified with a homomixer, whereby an O/W-type emulsion foundation was obtained.

### (Evaluation Methods for Sense of Use, Cosmetic Effect, and Cosmetic Durability)

The sense of use, cosmetic effect, and cosmetic durability were evaluated based on the average of scores given by 25 expert panelists who were asked to use each O/W-type emulsion foundation for one day and score on a five-point scale shown below. The sense of use was evaluated in terms of good smoothness, no stickiness, and comfort. Further, the cosmetic effect is evaluated in terms of powderiness, no uneven coating, uniformity of the cosmetic coat, and natural luster. Further, the cosmetic durability is evaluated in terms of occurrence of color dullness or shininess with the passage of time, and no powder aggregation.

### (Evaluation Criteria)

Evaluation result: score
Very good: 5 points
Good: 4 points
Average: 3 points
Slightly poor: 2 points
Poor: 1 point

**[Table 2]**

| | Components | Example 18 | Comparative Example 9 |
|---|---|---|---|
| Oil layer components | Isohexadecane | 13.0 (wt%) | 13.0 (wt%) |
| | Glyceryl tri-2-ethylhexanoate | 5.5 | 5.5 |
| | 2-Ethylhexyl p-methoxycinnamate | 5.0 | 5.0 |
| | Behenyl alcohol | 1.0 | 1.0 |
| | Dibutylhydroxytoluene | 0.05 | 0.05 |
| | Powder of Example 1 (titanium oxide) | 8.0 | - |
| | Powder of Example 2 (yellow iron oxide) | 3.1 | - |
| | Powder of Example 3 (red iron oxide) | 2.1 | - |
| | Powder of Example 4 (black iron oxide) | 0.2 | - |
| | Powder of Comparative Example 2 (titanium oxide) | - | 8.0 |
| | Powder of Comparative Example 3 (yellow iron oxide) | - | 3.1 |
| Aqueous layer components | Powder of Comparative Example 4 (red iron oxide) | - | 2.1 |
| | Powder of Comparative Example 5 (black iron oxide) | - | 0.2 |
| | BG | 5.0 | 5.0 |
| | Ethanol | 5.0 | 5.0 |
| | Carbomer | 0.2 | 0.2 |
| | Triethanolamine | 0.1 | 0.1 |
| | Phenoxyethanol | 0.5 | 0.5 |
| | Ion exchanged water | balance | balance |
| Evaluation results | Sense of use | 4.7 | 3.2 |
| | Cosmetic effect | 4.7 | 3.0 |
| | Cosmetic durability | 4.3 | 2.3 |

From the evaluation results of Example 18, it was found that even if the powders of Examples 1 to 4 are prepared into an O/W-type emulsion foundation, good sense of use, cosmetic effect, and cosmetic durability are obtained.

On the other hand, from the evaluation results of Comparative Example 9, it was found that when the powders of Comparative Examples 2 to 5 are prepared into an O/W-type emulsion foundation, the sense of use, cosmetic effect, and cosmetic durability are poorer than in the case where the powders of Examples 1 to 4 are used.

### [Evaluation of Sense of Use, Cosmetic Effect, and Cosmetic Durability of O/W-Type Emulsion Foundation 2]

O/W-type emulsion foundations having a composition shown in any of the following Example 19 and Comparative Examples 10 and 11 were prepared, and the sense of use, cosmetic effect, and cosmetic durability of each O/W-type emulsion foundation were evaluated.

### (Production Method and Evaluation Method)

The production method and evaluation method are the same as those in the above Example 18, but are significantly different in that comparison is made with Comparative Example 10 in which octyltriethoxysilane-treated hydrophobic pigment grade titanium oxide, octyltriethoxysilane-treated hydrophobic yellow iron oxide, octyltriethoxysilane-treated hydrophobic red iron oxide, and octyltriethoxysilane-treated hydrophobic black iron oxide (each corresponds to the hydrophobic inorganic powder before the hydrophilization treatment in Examples 1 to 4) were blended in the aqueous layer components.

**[Table 3]**

| | Components | Example 19 | Comparative Example 10 | Comparative Example 11 |
|---|---|---|---|---|
| | Decamethylcyclopentasiloxane | 11.0 (wt%) | 11.0 (wt%) | 11.0 (wt%) |
| | Isohexadecane | 5.5 | 5.5 | 5.5 |
| | Triethylhexanoin | 5.0 | 5.0 | 5.0 |
| | 2-Ethylhexyl p-methoxycinnamate | 8.0 | 8.0 | 8.0 |
| Oil layer components | PEG-9 polydimethylsiloxyethyl dimethicone | 4.0 | 4.0 | 4.0 |
| | Silicone-treated fine particle zinc oxide | 6.5 | 6.5 | 6.5 |
| | Octyltriethoxysilane-treated hydrophobic pigment grade titanium oxide | - | 7.5 | - |
| | Octyltriethoxysilane-treated hydrophobic yellow iron oxide | - | 3.0 | - |
| | Octyltriethoxysilane-treated hydrophobic red iron oxide | - | 1.2 | - |
| | Octyltriethoxysilane-treated hydrophobic black iron oxide | - | 0.3 | - |
| | Powder of Example 1 (titanium oxide) | 7.5 | - | - |
| | Powder of Example 2 (yellow iron oxide) | 3.0 | - | - |
| | Powder of Example 3 (red iron oxide) | 1.2 | - | - |
| | Powder of Example 4 (black iron oxide) | 0.3 | - | - |
| | Powder of Comparative Example 2 (titanium oxide) | - | - | 7.5 |
| Aqueous layer components | Powder of Comparative Example 3 (yellow iron oxide) | - | - | 3.0 |
| | Powder of Comparative Example 4 (red iron oxide) | - | - | 1.2 |
| | Powder of Comparative Example 5 (black iron oxide) | - | - | 0.3 |
| | BG | 6.0 | 6.0 | 6.0 |
| | Phenoxyethanol | 0.8 | 0.8 | 0.8 |
| | Ion exchanged water | to 100.0 | to 100.0 | to 100.0 |
| Evaluation results | Sense of use | 4.3 | 3.9 | 2.6 |
| | Cosmetic effect | 4.5 | 3.7 | 2.4 |
| | Cosmetic durability | 4.0 | 4.2 | 2.0 |

From the evaluation results of Example 19, it was found that when the powders of Examples 1 to 4 are blended as the aqueous layer components of the O/W-type emulsion foundation (Example 19), good sense of use, cosmetic effect, and cosmetic durability are obtained.

Further, from the evaluation results of Comparative Example 10, it was found that even when the hydrophobic inorganic powders before the hydrophilization treatment are blended as the oil layer components of the O/W-type emulsion foundation (Comparative Example 10), reasonable sense of use, cosmetic effect, and cosmetic durability are obtained, but the sense of use and cosmetic effect are poorer than in Example 19.

From the evaluation results of Comparative Examples 10 and 11, it was found that in the case where the powders of Comparative Examples 2 to 5 are used, the sense of use, cosmetic effect, and cosmetic durability are poorer than in the case where the powders before the hydrophilization treatment are used. That is, it was found that in the case where the treatment is performed with a nonionic surfactant having a linear carbon chain moiety, deterioration occurs in terms of sense of use, cosmetic effect, and cosmetic durability as compared with the case where the hydrophobic inorganic powders before the hydrophilization treatment are used. Note that Comparative Examples 10 and 11 are different in whether the powders are blended in the aqueous layer or the oil layer, but are interpreted as rational in consideration of the surface coat of each powder. For example, when the powders of Comparative Examples 2 to 5 (powders subjected to the hydrophilization treatment) are blended as the oil layer components, further deterioration in the sense of use, cosmetic effect, and cosmetic durability is expected.

### [Evaluation of Sense of Use, Cosmetic Effect, Cosmetic Durability, and SPF Value of Water-Based Suncut Lotion]

Water-based suncut lotions having a composition shown in any of the following Example 20 and Comparative Examples 12 and 13 were prepared, and the sense of use, cosmetic effect, cosmetic durability, and SPF value of each water-based suncut lotion was evaluated.

### (Production Method)

A: The oil layer components were well dispersed and mixed.
B: The aqueous layer components were well dispersed and mixed.
C: After A was added to B, the resultant was emulsified with a homomixer, whereby a water-based suncut lotion was obtained.

### (Evaluation Method)

For the water-based suncut lotion, the in-vitro SPF value was measured as an additional item. The other evaluation methods are the same as those for the above-mentioned O/W-type emulsion foundation, but are significantly different in that comparison is made with the example in which the hydrophobic inorganic powders before the hydrophilization treatment (each corresponds to the hydrophobic inorganic powder before the hydrophilization treatment in Examples 10 and 11) in Comparative Example 12 are blended in the aqueous layer components.

**[Table 4]**

| | Components | Exampl e 20 | Comparativ e Example 12 | Comparativ e Example 13 |
|---|---|---|---|---|
| Oil layer component s | Decamethylcyclopentasiloxan e | 15.0 (wt%) | 15.0 (wt%) | 15.0 (wt%) |
| | Dimethylpolysiloxane (6 cs) | 5.0 | 5.0 | 5.0 |
| | Triethylhexanoin | 6.0 | 6.0 | 6.0 |
| | Hydrogen dimethicone-treated hydrophobic black iron oxide | - | 8.0 | - |
| | Dimethylpolysiloxane and methyl hydrogen polysiloxane hybrid treated hydrophobic fine particle titanium oxide | - | 10.0 | - |
| | Powder of Example 10 (titanium oxide) | 8.0 | - | - |
| | Powder of Example 11 (zinc oxide) | 10.0 | - | - |
| | Powder of Comparative Example 6 (titanium oxide) | - | - | 8.0 |
| Aqueous layer component s | Powder of Comparative Example 7 (zinc oxide) | - | - | 10.0 |
| | PEG-10 dimethicone | 3.0 | 3.0 | 3.0 |
| | Glyceryl monostearate | 1.5 | 1.5 | 1.5 |
| | BG | 5.0 | 5.0 | 5.0 |
| | Ethanol | 5.0 | 5.0 | 5.0 |
| | Purified water | Balance | Balance | Balance |
| Evaluation results | Sense of use | 4.5 | 4.0 | 3.5 |
| | Cosmetic effect | 4.4 | 3.9 | 3.5 |
| | Cosmetic durability | 4.2 | 4.0 | 3.3 |
| | In-vitro SPF value | 38.2 | 33.6 | 27.0 |

From the results in Table 4, it was found that the water-based suncut lotion obtained by blending the powders of Examples 10 and 11 has a high UV shielding ability and excellent sense of use, cosmetic effect, and cosmetic durability.

### [Evaluation of Sense of Use, Cosmetic Effect, Cosmetic Durability, and SPF value of O/W-Type Sunscreen Cosmetic 1]

O/W-type sunscreen cosmetics having compositions shown in the following Example 21 and Comparative Example 14 were prepared, and the sense of use, cosmetic effect, cosmetic durability, and SPF value of each O/W-type sunscreen cosmetic were evaluated.

### (Production Method and Evaluation Method)

The production method and evaluation method for the O/W-type sunscreen cosmetic are the same as those for the water-based suncut lotion described above.

**[Table 5]**

| | Components | Example 21 | Comparative Example 14 |
|---|---|---|---|
| Oil layer components | Isododecane | 8.0 | 8.0 |
| | Glyceryl octanoate | 4.0 | 4.0 |
| | Dimethylpolysiloxane (10 cs) | 3.0 | 3.0 |
| | Cetostearyl alcohol | 1.0 | 1.0 |
| | 2-Ethylhexyl p-methoxycinnamate | 5.0 | 5.0 |
| | Powder of Example 11 (zinc oxide) | 12.0 | - |
| | Powder of Comparative Example 7 (zinc oxide) | - | 12.0 |
| | PEG-80 hydrogenated castor oil | 1.0 | 1.0 |
| | Acrylate/sodium acryloyldimethyltaurate copolymer | 0.2 | 0.2 |
| Aqueous layer components | Xanthan gum | 0.1 | 0.1 |
| | Phenoxyethanol | 0.7 | 0.7 |
| | Glycerin | 5.0 | 5.0 |
| | Ethanol | 5.0 | 5.0 |
| | Purified water | Balance | Balance |
| Evaluation results | Sense of use | 4.8 | 4.0 |
| | Cosmetic effect | 4.5 | 4.0 |
| | Cosmetic durability | 4.2 | 4.0 |
| | In-vitro SPF value | 31.7 | 25.2 |

From the results in Table 5, it was found that the O/W-type sunscreen cosmetic obtained by blending the powder of Example 7 has a high UV shielding ability and excellent sense of use, cosmetic effect, and cosmetic durability.

### [Evaluation of Sense of Use, Cosmetic Effect, Cosmetic Durability, and SPF value of O/W-Type Sunscreen Cosmetic 2]

O/W-type sunscreen cosmetics having a composition shown in either of the following Example 22 and Comparative Example 15 were prepared, and the sense of use, cosmetic effect, cosmetic durability, and SPF value of each O/W-type sunscreen cosmetic were evaluated.

### (Production Method and Evaluation Method)

The production method and evaluation method for the O/W-type sunscreen cosmetic are the same as those for the water-based suncut lotion described above.

**[Table 6]**

| | Components | Example 22 | Comparative Example 15 |
|---|---|---|---|
| Oil layer components | Decamethylcyclopentasiloxane | 10.0 (wt%) | 10.0 (wt%) |
| | Isododecane | 9.0 | 9.0 |
| | Diisopropyl sebacate | 8.0 | 8.0 |
| | PEG-10 dimethicone | 4.0 | 4.0 |
| | Diethylamino hydroxybenzoyl hexyl benzoate | 8.0 | 8.0 |
| Aqueous layer components | Powder of Example 10 (titanium oxide) | 5.0 | - |
| | Powder of Example 11 (zinc oxide) | 10.0 | - |
| | Powder of Comparative Example 6 (titanium oxide) | - | 5.0 |
| | Powder of Comparative Example 7 (zinc oxide) | - | 10.0 |
| | BG | 10.0 | 10.0 |
| | Erythritol | 2.0 | 2.0 |
| | Phenoxyethanol | 0.5 | 0.5 |
| | Purified water | Balance | Balance |
| Evaluation results | Sense of use | 4.5 | 4.2 |
| | Cosmetic effect | 4.3 | 3.9 |
| | Cosmetic durability | 4.3 | 3.9 |
| | In-vitro SPF value | 45.6 | 38.1 |

From the results in Table 6, it was found that the O/W-type sunscreen cosmetic obtained by blending the powders of Examples 10 and 11 has a high UV shielding ability and excellent sense of use, cosmetic effect, and cosmetic durability.

### [Evaluation of Sense of Use, Cosmetic Effect, and Cosmetic Durability of Powder Foundation]

Powder foundations having compositions shown in the following Example 23 and Comparative Example 16 were prepared, and the sense of use, cosmetic effect, and cosmetic durability of each powder foundation were evaluated.

### (Production Method)

A: The powder components were well dispersed and mixed.
B: The oily components were well mixed and dissolved.
C: After B was added to A, the resultant was mixed and pulverized, and then allowed to pass through a sieve and molded in a metal plate, whereby a powder foundation was obtained.

### (Evaluation Method)

The evaluation method for the powder foundation is the same as that for the O/W-type emulsion foundation described above.

**[Table 7]**

| | Components | Example 23 | Comparative Example 16 |
|---|---|---|---|
| | Silicone-treated talc | balance (wt%) | balance (wt%) |
| | Silicone-treated sericite | 16.0 | 25.0 |
| | Silicone-treated mica | 10.0 | 10.0 |
| | Silicone-treated spherical silica | 7.0 | 5.0 |
| | Powder of Example 6 (titanium oxide) | 8.0 | - |
| Powder components | Powder of Example 7 (yellow iron oxide) | 2.8 | - |
| | Powder of Example 8 (red iron oxide) | 1.3 | - |
| | Powder of Example 9 (black iron oxide) | 0.2 | - |
| | Powder of Comparative Example 2 (titanium oxide) | - | 8.5 |
| | Powder of Comparative Example 3 (yellow iron oxide) | - | 3.1 |
| | Powder of Comparative Example 4 (red iron oxide) | - | 2.0 |
| | Powder of Comparative Example 5 (black iron oxide) | - | 0.3 |
| Oily components | 2-Ethylhexyl p-methoxycinnamate | 3.0 | 3.0 |
| | Glyceryl tri-2-ethylhexanoate | 2.0 | 2.0 |
| | Dimethylpolysiloxane (20 cs) | 3.0 | 3.0 |
| | Squalene | 3.0 | 3.0 |
| | Sorbitan sesquioleate | 0.5 | 0.5 |
| | Antibacterial agent | q.S. | q.s. |
| | Antioxidant | q.S. | q.s. |
| Evaluation results | Sense of use | 4.6 | 3.9 |
| | Cosmetic effect | 4.6 | 3.7 |
| | Cosmetic durability | 4.2 | 3.9 |

From the results in Table 7, it was found that the powder foundation obtained by blending the powders of Examples 6 to 9 has excellent sense of use, cosmetic effect, and cosmetic durability.

### [Evaluation of Sense of Use, Cosmetic Effect, and Cosmetic Durability of Oily Solid Foundation]

Oily solid foundations having a composition shown in either of the following Example 24 and Comparative Example 17 were prepared, and the sense of use, cosmetic effect, and cosmetic durability of each oily solid foundation were evaluated.

### (Production Method)

A: The powder components were well dispersed and mixed.
B: The oily components were well mixed and dissolved.
C: After B was added to A, the resultant was treated with a heat roller and poured into a metal plate, and then cooled and molded, whereby an oily solid foundation was obtained.

### (Evaluation Method)

The evaluation method for the oily solid foundation is the same as that for the O/W-type emulsion foundation described above.

**[Table 8]**

| | Components | Example 24 | Comparative Example 17 |
|---|---|---|---|
| Oily components | Polyglyceryl-2 triisostearate | 8.5 (wt%) | 8.5 (wt%) |
| | Propylene glycol dicaprate | 10.0 | 10.0 |
| | Dimethylpolysiloxane (20 cs) | 9.0 | 9.0 |
| | (Dimethicone/vinyl dimethicone) crosspolymer | 5.0 | 5.0 |
| | Petrolatum | 7.5 | 7.5 |
| | Polyethylene wax | 4.0 | 4.0 |
| | Candelilla wax | 1.5 | 1.5 |
| | 2-Ethylhexyl p-methoxycinnamate | 3.0 | 3.0 |
| | Alkylsilane-treated talc | balance | balance |
| | Powder of Example 1 (titanium oxide) | 7.0 | - |
| | Powder of Example 2 (yellow iron oxide) | 3.0 | - |
| | Powder of Example 3 (red iron oxide) | 2.2 | - |
| Powder components | Powder of Example 4 (black iron oxide) | 0.2 | - |
| | Powder of Comparative Example 2 (titanium oxide) | - | 7.0 |
| | Powder of Comparative Example 3 (yellow iron oxide) | - | 3.0 |
| | Powder of Comparative Example 4 (red iron oxide) | - | 2.2 |
| | Powder of Comparative Example 5 (black iron oxide) | - | 0.2 |
| | Preservative | q.S. | q.s. |
| Evaluation results | Sense of use | 4.6 | 4.0 |
| | Cosmetic effect | 4.6 | 3.7 |
| | Cosmetic durability | 4.3 | 3.9 |

From the results in Table 8, it was found that the oily solid foundation obtained by blending the powders of Examples 1 to 4 has excellent sense of use, cosmetic effect, and cosmetic durability.

### [Evaluation of Sense of Use, Cosmetic Effect, and Cosmetic Durability of Water-Based White Powder Foundation]

Water-based white powder foundations having compositions shown in either of the following Example 25 and Comparative Example 18 were prepared, and the sense of use, cosmetic effect, and cosmetic durability of each water-based white powder foundation were evaluated.

### (Production Method)

A: The powder components were well mixed.
B: The aqueous layer components were mixed and dissolved.
C: After A was added to B, the resultant was well stirred, whereby a water-based white powder foundation was obtained.

### (Evaluation Method)

The evaluation method for the water-based white powder foundation is the same as that for the O/W-type emulsion foundation described above.

**[Table 9]**

| | Components | Example 25 | Comparative Example 18 |
|---|---|---|---|
| Powder components | Talc | 10.0 (wt%) | 10.0 (wt%) |
| | Boron nitride | 3.0 | 3.0 |
| | Synthetic mica | 3.5 | 3.5 |
| | Powder of Example 5 (titanium oxide) | 3.0 | - |
| | Powder of Comparative Example 1 (titanium oxide) | - | 3.0 |
| | Polyurethane powder | 5.0 | - |
| Aqueous layer components | BG | 5.0 | 5.0 |
| | Glycerin | 5.0 | 5.0 |
| | Ethanol | 5.0 | 5.0 |
| | EDTA·2Na | 0.2 | 0.2 |
| | Phenoxyethanol | 0.3 | 0.3 |
| | SEPINOV P88 | 0.2 | 0.2 |
| | Ion exchanged water | balance | balance |
| Evaluation results | Sense of use | 4.2 | 3.3 |
| | Cosmetic effect | 4.1 | 3.1 |
| | Cosmetic durability | 3.5 | 3.0 |

From the results in Table 9, it was found that the water-based white powder foundation obtained by blending the powder of Example 5 has excellent sense of use, cosmetic effect, and cosmetic durability.

### [Evaluation of Sense of Use, Cosmetic Effect, and Cosmetic Durability of Water-Based Eye Shadow Obtained by Wet Molding Method]

Water-based eye shadows having compositions shown in the following Example 26 and Comparative Example 19 were prepared, and the sense of use, cosmetic effect, and cosmetic durability of each water-based eye shadow were evaluated.

### (Production Method)

A: The powder components were well mixed.
B: The aqueous layer components were mixed and dissolved.
C: After A was added to the above B, the resultant was well stirred to form a slurry, which was poured into a filling dish and dried, whereby a water-based eye shadow was obtained.

### (Evaluation Method)

The evaluation method for the water-based eye shadow is the same as that for the O/W-type emulsion foundation described above.

**[Table 10]**

| | Components | Example 26 | Comparative Example 19 |
|---|---|---|---|
| | Polymethylsilsesquioxane | 5.0 (wt%) | 5.0 (wt%) |
| | Pearl pigment | 35.0 | 35.0 |
| | Hydrophilized hydrophobic titanium oxide of Example 1 | 5.5 | - |
| | Hydrophilized hydrophobic yellow iron oxide of Example 7 | 2.0 | - |
| Powder components | Hydrophilized hydrophobic red iron oxide of Example 8 | 0.9 | - |
| | Hydrophilized titanium oxide of Comparative Example 1 | - | 5.5 |
| | Hydrophilized yellow iron oxide of Comparative Example 3 | - | 2.0 |
| | Hydrophilized red iron oxide of Comparative Example 4 | - | 0.9 |
| | Hydrophilized hydrophobic talc of Example 17 | 51.6 | 51.6 |
| Aqueous layer components | Ethanol | 5.0 | 5.0 |
| | EDTA·2Na | 0.2 | 0.2 |
| | Phenoxyethanol | 0.3 | 0.3 |
| | Ion exchanged water | 144.5 | 144.5 |
| Evaluation results | Sense of use | 4.7 | 2.4 |
| | Cosmetic effect | 4.4 | 2.8 |
| | Cosmetic durability | 4.3 | 2.5 |

From the results in Table 10, it was found that the water-based eye shadow obtained by blending the powders of Examples 1,7, and 8 has excellent sense of use, cosmetic effect, and cosmetic durability.

### [Evaluation of Sense of Use, Cosmetic Effect, and Cosmetic Durability of Water-Based Makeup Base]

Water-based makeup bases having compositions shown in the following Example 27 and Comparative Example 20 were prepared, and the sense of use, cosmetic effect, and cosmetic durability of each water-based makeup base were evaluated.

### (Production Method)

A: The powder components were well mixed.
B: BG of the aqueous layer component and the component A were mixed and treated with a roller.
C: After A was added to B, the resultant was well stirred, whereby a water-based makeup base was obtained.

### (Evaluation Method)

The evaluation method for the water-based makeup base is the same as that for the O/W-type emulsion foundation described above.

**[Table 11]**

| | Components | Example 27 | Comparative Example 20 |
|---|---|---|---|
| Powder components | Silicone-treated talc | 7.0 (wt%) | 7.0 (wt%) |
| | Powder of Example 2 (yellow iron oxide) | 0.7 | - |
| | Powder of Example 3 (red iron oxide) | 0.3 | - |
| | Powder of Example 4 (black iron oxide) | 3.0 | - |
| | Powder of Comparative Example 3 (yellow iron oxide) | - | 0.7 |
| | Powder of Comparative Example 4 (red iron oxide) | - | 0.3 |
| | Powder of Comparative Example 5 (black iron oxide) | - | 3.0 |
| Aqueous layer components | BG | 10.0 | 10.0 |
| | Glycerin | 5.0 | 5.0 |
| | Ethanol | 9.0 | 9.0 |
| | EDTA·2Na | 0.2 | 0.2 |
| | Phenoxyethanol | 0.3 | 0.3 |
| | Ion exchanged water | balance | balance |
| Evaluation results | Sense of use | 4.5 | 3.1 |
| | Cosmetic effect | 4.5 | 3.0 |
| | Cosmetic durability | 4.5 | 2.4 |

From the results in Table 11, it was found that the water-based makeup base obtained by blending the powders of Examples 2 to 4 has excellent sense of use, cosmetic effect, and cosmetic durability.

### [Evaluation of Sense of Use, Cosmetic Effect, and Cosmetic Durability of Lipstick]

Lipsticks having a composition shown in either of the following Example 28 and Comparative Example 21 were prepared, and the sense of use, cosmetic effect, and cosmetic durability of each lipstick were evaluated.

### (Production Method)

A: The oil layer components were well mixed.
B: The powder components were mixed with the component A, and the resultant was subjected to a dispersion treatment with a roller.
C: After B was added to A, the resultant was uniformly mixed.
D: The aqueous layer components were mixed and heated.
E: After D was added to C, the resultant was emulsified, whereby a lipstick was obtained.

### (Evaluation Method)

The evaluation method for the lipstick is the same as that for the O/W-type emulsion foundation described above.

**[Table 12]**

| | Components | Example 28 | Comparative Example 21 |
|---|---|---|---|
| Oil layer components | Dextrin palmitate/ethylhexanoate | 8.0 (wt%) | 8.0 (wt%) |
| | Cetyl octanoate | 20.0 | 20.0 |
| | PEG-10 dimethicone | 3.0 | 3.0 |
| | Decamethylcyclopentasiloxane | 40.0 | 40.0 |
| Powder components | Bentonite | 0.8 | 0.8 |
| | Powder of Example 5 (titanium oxide) | 3.5 | - |
| | Powder of Comparative Example 1 (titanium oxide) | - | 3.5 |
| | Powder of Example 3 (red iron oxide) | 0.7 | - |
| | Powder of Comparative Example 4 (red iron oxide) | - | 0.7 |
| Aqueous layer components | BG | 5.0 | 5.0 |
| | Sodium chloride | 0.5 | 0.5 |
| | Purified water | balance | balance |
| Evaluation results | Sense of use | 4.0 | 4.0 |
| | Cosmetic effect | 4.6 | 3.8 |
| | Cosmetic durability | 4.3 | 4.0 |

From the results in Table 12, it was found that the lipstick obtained by blending the powders of Examples 3 and 5 has excellent sense of use, cosmetic effect, and cosmetic durability.

### [Evaluation of No Stickiness, No Oily Sensation, and Comfort of Antiperspirant]

Antiperspirants having compositions shown in the following Example 29 and Comparative Example 22 were prepared, and the no stickiness, no oily sensation, and comfort of each antiperspirant were evaluated.

### (Production Method)

A: The powder components were well mixed.
B: The aqueous layer components were mixed and dissolved.
C: After A was added to B, the resultant was mixed, whereby an antiperspirant was obtained.

### (Evaluation Method)

The evaluation method for the antiperspirant is the same as that for the O/W-type emulsion foundation described above except that the antiperspirant was evaluated for no stickiness, no oily sensation, and comfort. However, in Comparative Example 22, talc was used as the inorganic powder. The talc as the inorganic powder is untreated talc which does not have a hydrophobic coat or a hydrophilic coat.

**[Table 13]**

| | Components | Example 29 | Comparative Example 22 |
|---|---|---|---|
| Powder components | Powder of Example 16 (zinc oxide) | 2.5 (wt%) | - |
| | Powder of Example 17 (talc) | 6.0 | - |
| | Powder of Comparative Example 7 (zinc oxide) | - | 2.5 (wt%) |
| | Talc as inorganic powder | - | 6.0 |
| | Silica beads | 5.0 | 5.0 |
| Aqueous layer components | Sodium chloride | 0.1 | 0.1 |
| | Ethanol | 38.0 | 38.0 |
| | BG | 2.0 | 2.0 |
| | Polyoxyethylene sorbitan monolaurate | 0.2 | 0.2 |
| | Phenoxyethanol | 0.3 | 0.3 |
| | Ion exchanged water | balance | balance |
| Evaluation results | No stickiness | 4.3 | 3.7 |
| | No oily sensation | 4.5 | 3.8 |
| | Comfort | 4.4 | 2.6 |

From the results in Table 13, it was found that the antiperspirant obtained by blending the powders of Examples 16 and 17 has no stickiness and no oily sensation, and has excellent comfort.

### [Evaluation of No Stickiness, Hair Combability, and Hair Smoothness of Hair Treatment]

Hair treatments having a composition shown in either of the following Example 30 and Comparative Example 23 were prepared, and the no stickiness, hair combability, and hair smoothness of each hair treatment were evaluated. The no stickiness, hair combability, and hair smoothness can also be reworded as luster and silkiness.

### (Production Method)

A: The oil layer components were heated and mixed.
B: The aqueous layer components were dispersed and mixed.
C: After B was added to A, the resultant was well mixed, whereby a hair treatment was obtained.

### (Evaluation Method)

The evaluation method for the hair treatment is the same as that for the O/W-type emulsion foundation described above except that the hair treatment was evaluated for no stickiness, hair combability, and hair smoothness. However, in Comparative Example 23, talc was used as the inorganic powder. The talc as the inorganic powder is untreated talc which does not have a hydrophobic coat or a hydrophilic coat.

**[Table 14]**

| | Components | Example 30 | Comparative Example 23 |
|---|---|---|---|
| Oil layer components | Ethylene glycol distearate | 1.5 (wt%) | 1.5 (wt%) |
| | Liquid paraffin | 10.0 | 10.0 |
| | Squalene | 5.0 | 5.0 |
| | Stearyl alcohol | 1.5 | 1.5 |
| | Dimethylpolysiloxane (10 cs) | 3.5 | 3.5 |
| | Stearic acid | 5.0 | 5.0 |
| Aqueous layer components | Powder of Example 17 (talc) | 5.0 | - |
| | Talc as inorganic powder | - | 5.0 |
| | Polyoxyethylene (3) stearyl alcohol | 4.5 | 4.5 |
| | Polyoxyethylene (10) cetyl ether | 2.0 | 2.0 |
| | BG | 6.0 | 6.0 |
| | Preservative | q.S. | q.s. |
| | Purified water | balance | balance |
| Evaluation results | No stickiness upon use | 4.3 | 3.8 |
| | Hair combability | 4.3 | 3.7 |
| | Hair smoothness | 4.5 | 3.6 |

From the results in Table 14, it was found that the hair treatment obtained by blending the powder of Example 17 has no stickiness and has excellent hair combability and hair smoothness.

### [Conclusion]

As described above, in the hydrophilized inorganic powder including an inorganic powder as a base material, a hydrophobic coat that covers the surface of the inorganic powder, and a hydrophilic coat that covers the hydrophobic coat, as the composition of the hydrophilic coat that can achieve good hydrophilicity and self-dispersibility, a nonionic surfactant(s) that is (are) hydrophilic and has a branched structure in a carbon chain moiety can be selected. In particular, when any one of a condition that the number of carbon atoms in the carbon chain moiety is 12 to 20 or a condition that the HLB is 9.5 to 15.5 is met, good hydrophilicity and self-dispersibility can be achieved. In particular, as shown in FIGS. 1 and 2, the self-dispersibility is particularly remarkable, and it can be said that the properties cannot be achieved by known hydrophilized inorganic powders.

Such findings can be said to be particularly useful in the light of the fact that as shown in Comparative Examples 11 and 13, when a hydrophobic inorganic powder is subjected to a hydrophilization treatment non-selectively using a nonionic surfactant, there is a possibility that deterioration rather occurs in view of desired properties (sense of use, cosmetic effect, and cosmetic durability).

## Claims

1. A hydrophilized inorganic powder, comprising an inorganic powder as a base material, a hydrophobic coat that covers the surface of the inorganic powder, and a hydrophilic coat that covers the hydrophobic coat, wherein
the hydrophilic coat has a composition being a nonionic surfactant(s) having a hydrophilic moiety and a carbon chain moiety,
the carbon chain moiety of the nonionic surfactant(s) has a branched structure sufficient for imparting self-dispersibility to the inorganic powder having hydrophobicity,
the nonionic surfactant(s) is/are one or more selected from the following materials:
polyoxyethylene (10) isostearyl ether;
polyoxyethylene (10) isododecyl ether,
polyoxyethylene (12) isostearate;
polyoxyethylene (8) glyceryl isostearate;
polyoxyethylene (20) glyceryl triisostearate;
polyoxyethylene (20) octyldodecyl ether;
polyoxyethylene (5) hexyldecyl ether; and
polyglyceryl (10) monoisostearate, and
the composition of the hydrophobic coat is one or more selected from octyltriethoxysilane, disodium stearoyl glutamate, hydrogen dimethicone, dimethylpolysiloxane, and methyl hydrogen polysiloxane.

2. A cosmetic, comprising the hydrophilized inorganic powder according to claim 1 .

## Patentansprüche

1. Hydrophilisiertes anorganisches Pulver, umfassend ein anorganisches Pulver als Basismaterial, einen hydrophoben Überzug, der die Oberfläche des anorganischen Pulvers bedeckt, und einen hydrophilen Überzug, der den hydrophoben Überzug bedeckt, wobei
der hydrophile Überzug eine Zusammensetzung aufweist, bei der es sich um ein nichtionisches Tensid oder nichtionische Tenside mit einer hydrophilen Gruppierung und einer Kohlenstoffkettengruppierung handelt,
die Kohlenstoffkettengruppierung des nichtionischen Tensids bzw. der nichtionischen Tenside eine verzweigte Struktur aufweist, die ausreicht, um dem anorganischen Pulver mit Hydrophobie Selbstdispergierbarkeit zu verleihen,
es sich bei dem nichtionischen Tensid bzw. den nichtionischen Tensiden um eines oder mehrere handelt, die aus den folgenden Substanzen ausgewählt sind:
Polyoxyethylen-(10)-isostearylether;
Polyoxyethylen-(10)-isododecylether,
Polyoxyethylen-(12)-isostearat;
Polyoxyethylen-(8)-glycerylisostearat;
Polyoxyethylen-(20)-glyceryltriisostearat;
Polyoxyethylen-(20)-octyldodecylether;
Polyoxyethylen-(5)-hexyldecylether und
Polyglyceryl-(10)-monoisostearat, und
es sich bei der Zusammensetzung des hydrophoben Überzugs um eine oder mehrere handelt, die aus Octyltriethoxysilan, Dinatriumstearoylglutamat, Hydrogen Dimethicone, Dimethylpolysiloxan und Methylhydrogenpolysiloxan ausgewählt ist bzw. sind.

2. Kosmetikum, umfassend das hydrophilisierte anorganische Pulver nach Anspruch 1.

## Revendications

1. Poudre inorganique rendue hydrophile, comprenant une poudre inorganique comme matériau de base, un revêtement hydrophobe qui recouvre la surface de la poudre inorganique, et un revêtement hydrophile qui recouvre le revêtement hydrophobe, dans laquelle
le revêtement hydrophile a une composition qui est un ou plusieurs tensioactifs non ioniques ayant un groupement hydrophile et un groupement de chaîne carbonée,
le groupement de chaîne carbonée du ou des tensioactifs non ioniques a une structure ramifiée suffisante pour conférer une autodispersibilité à la poudre inorganique ayant une hydrophobicité,
le ou les tensioactifs non ioniques sont un ou plusieurs des matériaux suivants :
éther isostéarylique de polyoxyéthylène (10) ;
éther isododécylique de polyoxyéthylène (10),
isostéarate de polyoxyéthylène (12) ;
isostéarate de glycéryle de polyoxyéthylène (8) ;
triisostéarate de glycéryle de polyoxyéthylène (20) ;
éther octyldodécylique de polyoxyéthylène (20) ;
éther hexyldécylique de polyoxyéthylène (5) ; et
monoisostéarate de polyglycéryle (10), et
la composition du revêtement hydrophobe est un ou plusieurs composés choisis parmi l'octyltriéthoxysilane, le stéaroylglutamate disodique, l'hydrogéno-diméthicone, le diméthylpolysiloxane et le méthylhydrogéno-polysiloxane.

2. Produit cosmétique, comprenant la poudre inorganique rendue hydrophile selon la revendication 1.
